# EUROPÄISCHE PATENTSCHRIFT

(11) **EP 1 971 890 B1**
(45) Veröffentlichungstag und Bekanntmachung des Hinweises auf die Patenterteilung: **14.07.2010**
(21) Anmeldenummer: 06829347.1
(22) Anmeldetag: 06.12.2006
(51) Int. Cl.: G02B 21/22

(54) **VERFAHREN ZUM BETREIBEN EINES OPTISCHEN SYSTEMS UND OPTISCHES SYSTEM**
METHOD FOR OPERATING AN OPTICAL SYSTEM AND OPTICAL SYSTEM
PROCÉDÉ D'UTILISATION D'UN SYSTÈME OPTIQUE ET SYSTÈME OPTIQUE

(30) Priorität: 08.12.2005 EP 05026775
(43) Veröffentlichungstag der Anmeldung: 24.09.2008
(73) Patentinhaber: Swiss Medical Technology GmbH, 9443 Widnau (CH)
(72) Erfinder: LUBER, Joachim, CH-9430 St. Margrethen (CH)
(74) Vertreter: Zwicker, Jörk
(86) Internationale Anmeldenummer: PCT/EP2006/011718
(87) Internationale Veröffentlichungsnummer: WO 2007/065660

(56) Entgegenhaltungen:
- WO-A-02/056085
- DE-A1- 10 323 629
- DE-A1- 19 541 237
- DE-A1- 19 822 256
- US-A- 5 266 791
- US-B1- 6 268 957

## Beschreibung

Die Erfindung betrifft ein Verfahren zum Betreiben eines optischen Systems und ein optisches System.

### Hintergrund der Erfindung

Optische Systeme, beispielsweise Stereomikroskope, werden in verschiedensten Anwendungen dazu genutzt, eine räumliche Abbildung eines Untersuchungsobjektes zu erzeugen. Beispielsweise werden solche optischen Systeme in medizinischen Operationsmikroskopen eingesetzt, um einen Operationsbereich einzusehen.

Die räumliche Abbildung des Untersuchungsobjektes wird unter Verwendung des Stereomikroskops erzeugt, bei dem es sich um einen Vertreter aus der Geräteklasse von Auflichtmikroskopen handelt. Das Untersuchungsobjekt wird von außen beleuchtet. Es sind üblicherweise zwei getrennte Strahlengänge gebildet, die ihrerseits mit einem jeweils zugeordneten Linsensystem ausgestattet sind. Die optischen Achsen der beiden Strahlengänge verlaufen durch eine den Linsensystemen in Richtung des Untersuchungsobjektes vorgelagerte Sammellinsenanordnung. Sowohl die Linsensysteme als auch die Sammellinsenanordnung können mehrere optische Linsen umfassen. Die verschiedenen optischen Linsen werden genutzt, um einen Fokus sowie einen Zoom-Faktor des Stereomikroskops einzustellen. Zu diesem Zweck werden die Linsen einzeln oder in Gruppen entlang der jeweils zugeordneten optischen Achse verschoben.

Aus der Patentschrift US 6,268,957 B1 ist ein Stereomikroskop mit zwei Strahlengängen bekannt. Das offenbarte Stereomikroskop umfasst zwei Linsenanordnungen, die motorgesteuert bewegt werden können und die eine computergesteuerte Änderung des Zoomfaktors und der Fokuseinstellung ermöglichen.

Die Verschiebung optischer Elemente zur Fokuseinstellung oder zur Einstellung des Zoom-Faktors kann mit Hilfe motorgetriebener Verstelleinrichtungen ausgeführt werden, die das optische Element in eine gewünschte Position entlang der optischen Achse verlagern. Im Gegensatz zu der auch bekannten Verlagerung optischer Elemente per Hand werden solche motorgetriebenen Verstelleinrichtungen mit einem Betätigungsmittel, beispielsweise einer die Spannungs- oder Stromversorgung regelnden Steuerung, vom Benutzer betätigt. Solange der Benutzer das Betätigungsmittel einschaltet, verschiebt sich die Linse entlang der optischen Achse. Diese Bewegung stoppt der Benutzer, indem das Betätigungsmittel ausgeschaltet wird.

In dem Dokument DE 103 23 629 A1 ist ein Wanderfeld-Linearmotor beschrieben, mit dem in einer Hülse angeordnete optische Elemente, beispielsweise eine optische Linse, entlang der optischen Achse verschoben werden können. Dieses wird erreicht mittels eines entlang der optischen Achse bewegten magnetischen Wanderfeldes. Das magnetische Wanderfeld und somit auch die Verschiebestellungen der optischen Elemente sind sehr fein einstellbar. Der bekannte Wanderfeld-Linearmotor unterstützt die Miniaturisierung von optischen Systemen.

### Zusammenfassung der Erfindung

Aufgabe der Erfindung ist es, ein Verfahren zum Betreiben eines optischen Systems mit einem Mikroskop sowie ein optisches System zu schaffen, bei denen die Bedienfreundlichkeit verbessert ist, insbesondere hinsichtlich einer einfachen Bedienung und einer gezielten Positionierung der optischen Elemente innerhalb eines optischen Kanals.

Diese Aufgabe wird erfindungsgemäß durch ein Verfahren zum Betreiben eines optischen Systems mit einem Mikroskop nach dem unabhängigen Anspruch 1 sowie ein optisches System mit einem Mikroskop nach dem unabhängigen Anspruch 7 gelöst.

Die Erfindung umfaßt den Gedanken, ein optisches System so zu betreiben, daß als Reaktion auf eine Benutzereingabe zum Verändern einer Fokuseinstellung und eines Zoom-Faktors des Mikroskops eine gewünschte Fokuseinstellung und ein gewünschter Zoom-Faktor mit Hilfe von motorgetriebenen Verstelleinrichtungen automatisch erzeugt werden, indem als Reaktion auf die erfaßte Benutzereingabe in einer Steuereinrichtung Steuersignale entsprechend gespeicherten Vorabeinstellungen erzeugt und mit Hilfe der jeweiligen motorgetriebenen Verstelleinrichtungen in Bewegungen der optischen Elemente des Mikroskops umgesetzt werden. Zu diesem Zweck umfassen die gespeicherten Vorabeinstellungen elektronische Informationen über Positionen von optischen Elementen, insbesondere Linsen, des Mikroskops für bestimmte Fokuseinstellungen und Zoom-Faktoren. Auf diese Weise kann in Abhängigkeit von der erfaßten Benutzereingabe das Mikroskop unter Rückgriff auf die gespeicherten Vorabeinstellungen automatisch so eingestellt werden, daß eine bestimmte Fokuseinstellung und ein bestimmter Zoom-Faktor gegeben wird. Die Vorabeinstellungen sind für das optische System vorab ermittelt worden, beispielsweise mit Hilfe von Messungen auf einer optischen Bank, und in Form elektronischer Daten gespeichert.

Der Benutzer muß nicht die optischen Elemente selbständig entlang der optischen Achse verschieben und eine optimale Positionierung suchen, wie dies im Stand der Technik vorgesehen ist. Vielmehr gibt der Benutzer lediglich eine gewünschte Fokuseinstellung und einen gewünschten Zoom-Faktor vor, woraufhin das optische System selbsttätig unter Berücksichtigung der gespeicherten Vorabeinstellungen eine Fokus- / Zoom-Faktoreinstellung vornimmt. Es ist auch keine Verschiebung der Linsen von Hand notwendig.

Die Erfindung sieht vor, daß beim Einstellen der Fokuseinstellung eine grobe Fokuseinstellung ausgeführt wird, indem von den Steuersignalen umfaßte Grobsteuersignale in der Steuereinrichtung erzeugt und von der Steuereinrichtung an die motorgetriebene Verstelleinrichtung der Sammellinsenanordnung übertragen werden und die Sammellinsenanordnung mit Hilfe der motorgetriebenen Verstelleinrichtung der Sammellinsenanordnung den Grobsteuersignalen entsprechend verlagert wird. Auf unkomplizierte Weise ist so eine separate Grobeinstellung des Fokus ermöglicht, wahlweise als Vorstufe für eine anschließende Feinabstimmung des Fokus.

Die Erfindung sicht weiter vor, daß beim Einstellen der Fokuseinstellung eine feine Fokuseinstellung ausgeführt wird, indem von den Steuersignalen umfaßte Feinsteuersignale in der Steuereinrichtung erzeugt und von der Steuereinrichtung an die jeweilige motorgetriebene Verstelleinrichtung der ersten Linsenanordnung in einem oder allen Strahlengängen übertragen werden und die erste Linsenanordnung in einem oder allen Strahlengängen mit Hilfe der jeweiligen motorgetriebenen Verstelleinrichtung den Feinsteuersignalen entsprechend verlagert wird. Hierdurch ist eine optimierte Fokuseinstellung mittels einer feinen Abstufung der Linsenpositionierung ermöglicht.

Es ist weiter erfindungsgemäß vorgesehen, daß das Einstellen des Zoom-Faktors ausgeführt wird, indem von den Steuersignalen umfaßte Zoomsteuersignale in der Steuereinrichtung erzeugt und von der Steuereinrichtung an die jeweilige motorgetriebene Verstelleinrichtung der zweiten Linsenanordnung in einem oder allen Strahlengängen übertragen werden und die zweite Linsenanordnung in einem oder allen Strahlengängen mit Hilfe der jeweiligen motorgetriebenen Verstelleinrichtung den Zoomsteuersignalen entsprechend verlagert wird. Hierdurch ist eine für den Benutzer auf einfache Weise vorgebbare Einstellung eines gewünschten Zoom-Faktors ermöglicht. Auch im Umgang mit komplexen optischen Systemen ungeübte Benutzer können so ohne weiteres einen vorgegebenen Zoom-Faktor einstellen.

Der Umfang der zu speichernden elektronischen Informationen für die Vorabeinstellungsdaten wird bei einer zweckmäßigen Ausführungsform der Erfindung dadurch minimiert, daß beim Erzeugen der Steuersignale zum Einstellen der Fokuseinstellung und / oder des Zoom-Faktors in der Steuereinrichtung eine Interpolation zwischen ausgewählten Vorabeinstellungsdaten der gespeicherten Vorabeinstellungsdaten ausgeführt wird. Mittels Interpolation können auch Fokuseinstellungen und / oder Einstellungen von Zoom-Faktoren ausgeführt werden, für die die zugehörige Positionierung der Linsen nicht vorab gemessen wurde. Üblicherweise wird eine lineare Interpolation zwischen den herangezogenen Vorabeinstellungsdaten durchgeführt.

Bei einer zweckmäßigen Weiterbildung der Erfindung kann vorgesehen sein, daß die Sammellinsenanordnung, die erste Linsenanordnung und / oder die zweite Linsenanordnung beim Verlagern entsprechend der Steuersignale mit Hilfe der jeweiligen motorgetriebenen Verstelleinrichtung linear verschoben werden. Zur Ausführung des Verlagerns wird bevorzugt ein Linearmotor mit den Steuersignalen beaufschlagt. Eine derartige Bewegungsgestaltung beim Verlagern hat den Vorteil, daß eine feinstufige Verschiebung der Linsen ausgeführt werden kann. Dieses erhöht die Exaktheit der gewünschten Fokuseinstellung und / oder des gewünschten Zoom-Faktors.

Um die räumliche Abbildung des Untersuchungsobjektes für eine weitergehende Bearbeitung zur Verfügung zu stellen, ist bei einer vorteilhaften Ausgestaltung der Erfindung vorgesehen, daß das Untersuchungsobjekt auf mehrere elektronische Speichermedien, die jeweils einem Strahlengang zugeordnet sind, räumlich abgebildet wird. Ebenso können natürlich die optischen Strahlengänge direkt, über so genannte Tuben, den Augen des Betrachters zur Verfügung gestellt werden.

Die Erfindung sieht ferner vor, daß in den Strahlengängen ein jeweiliger Zoom-Faktor eingestellt wird, indem die zweite Linsenanordnung mit Hilfe der motorgetriebenen Verstelleinrichtung verlagert wird, wobei sich die jeweils einstellbaren Zoom-Faktoren der Strahlengänge voneinander unterscheiden. Auf diese Weise ist es ermöglicht, eine vergrößerte Anzeige eines der Kanäle auf einem Monitor oder einer Fotokamera zu erzeugen, wohingegen der andere Kanal eine geringere Vergrößerung aufweist, beispielsweise für die visuelle Betrachtung des Operateurs, wenn das optische System als ein Stereomikroskop ausgeführt ist.

Das optische System umfaßt vorzugsweise ein Stereomikroskop.

Als besonders vorteilhaft für eine exakte und fein abgestufte Verlagerung von Linsen in dem Mikroskop haben sich motorgetriebene Verstelleinrichtungen unter Verwendung eines bewegten magnetischen Wanderfeldes erwiesen, wie es beispielsweise in dem Dokument DE 103 23 629 A1 beschrieben ist.

Die Ausgestaltungen der Erfindung in den abhängigen Unteransprüchen zu dem optischen System mit einem Mikroskop weisen die in Verbindung mit den zugehörigen Merkmalen in den abhängigen Verfahrensansprüchen erläuterten Vorteile entsprechend auf.

### Beschreibung bevorzugter Ausführungsbeispiele der Erfindung

Die Erfindung wird im folgenden anhand von Ausführungsbeispielen unter Bezugnahme auf die Zeichnungen näher erläutert. Hierbei zeigen:
- Fig. 1: eine schematische Darstellung eines optischen Systems mit einem Stereo- mikroskop in Draufsicht; und
- Fig. 2: eine perspektivische Darstellung von optischen Linsen des Stereomikros- kops in dem optischen System nach Fig. 1.

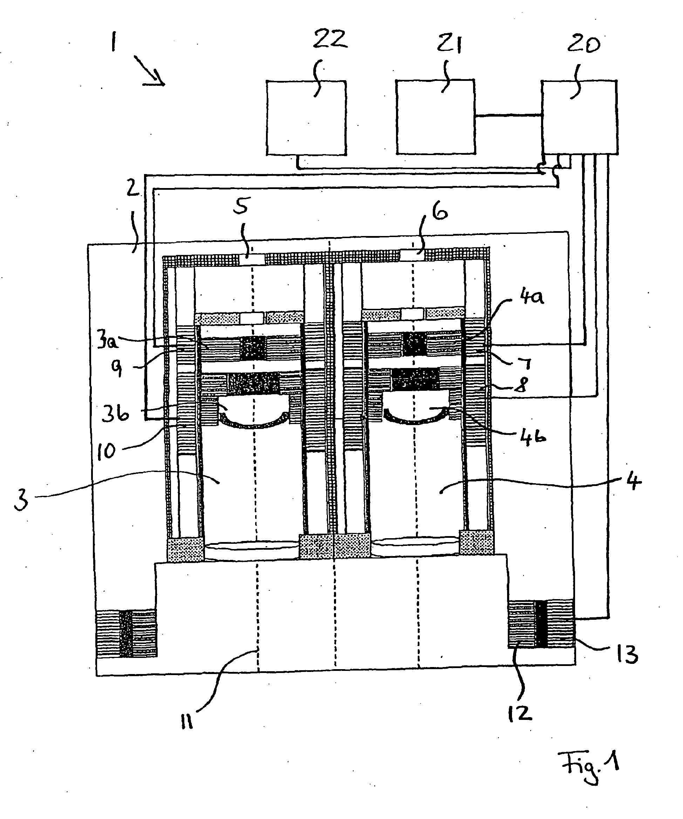

Fig. 1 zeigt eine schematische Darstellung eines optischen Systems 1 mit einem Stereomikroskop 2. In dem Stereomikroskop 2 sind zwei Strahlengänge 3, 4 gebildet. Mit Hilfe des Stereomikroskops 2 wird ein Untersuchungsobjekt (nicht dargestellt) unter Nützung der beiden Strahlengänge 3, 4 auf Videochips 5, 6 räumlich abgebildet. Die Videochips 5, 6 dienen als elektronische Speicher für die räumliche Abbildung, so daß anschließend eine Bildbearbeitung ausgeführt werden kann.

Entlang der optischen Achse in den beiden Strahlengängen 3, 4 sind jeweils eine erste und eine zweite Linsenanordnung 3a, 3b; 4a, 4b angeordnet. Die erste und die zweite Linsenanordnung 3a, 3b; 4a, 4b umfassen jeweils eine oder mehrere optische Linsen. Die optischen Linsen sind in einer jeweiligen Aufnahme gehalten, die ihrerseits mittels motorgetriebener Verstellglieder 7, 8, 9, 10 entlang der jeweiligen optischen Achse verlagerbar sind.

Den beiden Strahlengängen 3, 4 ist eine Sammellinsenanordnung 11 zugeordnet, die ebenfalls in einer Aufnahme 12 gehalten wird. In der Aufnahme 12 ist die Sammellinsenanordnung 11 mit Hilfe eines weiteren Verstellgliedes 13 verschiebbar.

Die motorgetriebenen Verstellglieder 7, 8, 9, 10, 13 sind an eine Steuereinrichtung 20 gekoppelt, so daß von der Steuereinrichtung 20 erzeugte Steuersignale an die motorgetriebenen Verstellglieder 7, 8, 9, 10, 13 übertragen werden können, um eine Fokuseinstellung und / oder eine Einstellung eines Zoom-Faktors des Stereomikroskops 2 vorzunehmen. Die Steuereinrichtung 20 ihrerseits ist mit einer Eingabeeinrichtung 21 verbunden, mit der Benutzereingaben erfaßt werden. Bei der Eingabeeinrichtung 21 handelt es sich beispielsweise um ein Tastfeld oder eine Tastatur, wie dieses im Rahmen von Bedienfeldern von Geräten bekannt ist.

Die motorgetriebenen Verstellglieder 7, 8, 9, 10, 13 können drehfeldgetriebene Verstelleinrichtungen sein. Alternativ kann es sich bei den motorgetriebenen Verstellgliedern 7, 8, 9, 10, 13 um elektromagnetische Wanderfeld-Linearmotoren zur Bewegung der optischen Elemente handeln. Ein derartiger elektromagnetischer Wanderfeld-Linearmotor weist eine axial bewegliche Gleithülse auf, die in einem Hüllrohr gleitet und mindestens einen axial polarisierten Permanentmagneten sowie das optische Element bzw. die optischen Elemente aufnimmt. Außerdem ist eine Anordnung von mindestens drei Spulen vorgesehen, die um das Hüllrohr geschlungen sind und durch unabhängige, variable Bestromung ein magnetisches Wanderfeld erzeugen können, das durch einen magnetischen Rückfluss über ein weichmagnetisches Außenrohr und weichmagnetische Läuferpolschuhe konzentriert geführt und verstärkt wird. Das dreiphasige Wanderfeld dient zur axialen Bewegung des Permanentmagneten und der mit diesem verbundenen Gleithülse. Das Wanderfeld erzeugt durch Wechselwirkung mit dem Permanentmagneten Selbsthaltekräfte, die zur Festigung des Läuferorts sowie zur Positionsstabilisierung der optischen Elemente durch rücktreibende Kräfte führen. Die Verwendung von elektromagnetischen Wanderfeld-Linearmotoren als erfindungsgemäße motorgetriebene Verstelleinrichtungen ermöglicht eine äußerst kompakte Bausweise des erfindungsgemäßen optischen Systems 1. Für die weiteren Details derartiger elektromagnetischer Wanderfeld-Linearmotoren wird auf die Druckschrift DE 103 23 629 A1 verwiesen.

Die Baulänge des Stereomikroskops 2 ist dadurch reduziert, daß zur feinen Fokuseinstellung nicht nur die Sammellinsenanordnung 11 mittels des motorgetriebenen Verstellgliedes 13 bewegt wird, sondern auch die motorgetriebene Verstelleinrichtung 3a, 4a. Ein Zoom-Faktor des Stereomikroskops 2 wird mittels Verschieben der zweiten Linsenanordnung 3b, 4b erreicht. Die zweiten Linsenanordnungen 3b, 4b sind unabhängig voneinander einstellbar, so daß zu einem bestimmten Zeitpunkt in den beiden Strahlengängen 3, 4 voneinander abweichende Zoom-Faktoren eingestellt werden können.

Wenn mit Hilfe der Eingabeeinrichtung 21 eine Benutzereingabe zum Einstellen der Fokuseinstellung und / oder des Zoom-Faktors des Stereomikroskops 2 erfaßt wird, werden anschließend in der Steuereinrichtung 20 Steuersignale entsprechend Vorabeinstellungsdaten erzeugt, die in einer mit der Steuereinrichtung 20 verbundenen Speichereinrichtung 22 elektronisch gespeichert sind. Es handelt sich hierbei um elektronische Informationen betreffend die Positionierung der ersten Linsenanordnung 3a, 4a und der zweiten Linsenanordnung 3b, 4b in einem der beiden Strahlengängen 3, 4 und / oder der Sammellinsenanordnung 11 für bestimmte Fokuseinstellungen und / oder Zoom-Faktoren.

Wenn sich nach dem Erfassen der Benutzereingabe ergibt, daß zu der gewünschten Fokuseinstellung und / oder dem gewünschten Zoom-Faktor keine exakt passenden Voreinstellungsdaten gespeichert sind, ermittelt die Steuereinrichtung 20 Voreinstellungsdaten für benachbarte Fokuseinstellungen / Zoom-Faktoren und interpoliert hieraus linear die Informationen zum Erzeugen der entsprechenden Steuersignale.

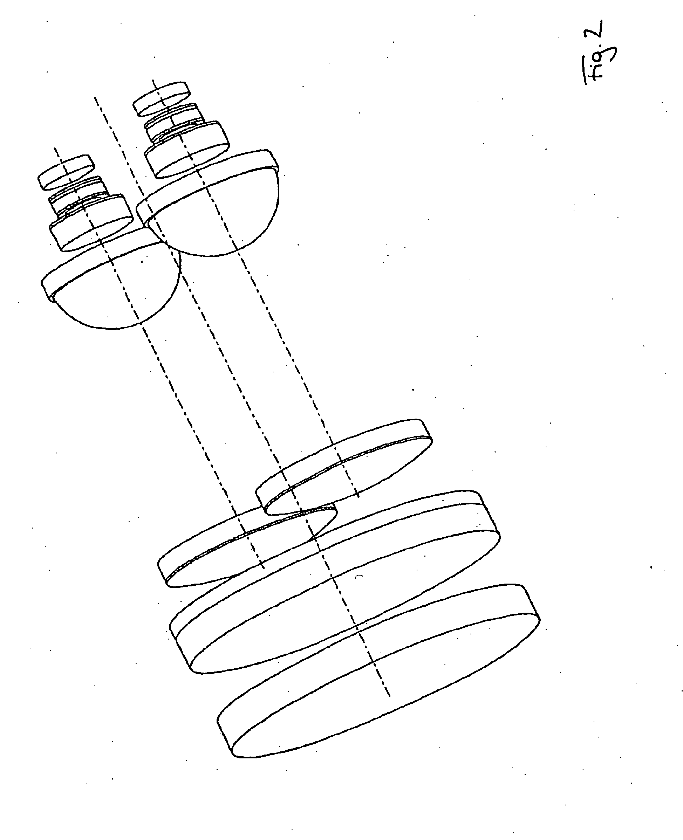

Fig. 2 zeigt eine perspektivische Darstellung von optischen Linsen der ersten und der zweiten Linsenanordnung 3a, 4a; 3b, 4b des Stereomikroskops 20 in dem optischen System nach Fig. 1.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Verfahren zum Betreiben eines optischen Systems (1) bei dem mittels eines von dem optischen System (1) umfassten stereomikroskops (2) ein Untersuchungsobjekt in eine Bildebene räumlich abgebildet werden kann, wobei das stereomikroskop (2) mindestens ein Linsensystem, welches in einem jeweiligen
Strahlengang (3, 4) angeordnet ist, und eine in den Strahlengängen (3, 4) angeordnete Sammellinsenanordnung (11) aufweist, wobei das mindestens eine Linsensystem eine erste Linsenanordnung (3a; 4a) und eine zwischen der ersten Linsenanordnung (3a; 4a) und der Sammellinsenanordnung (11) angeordnete, zweite Linsenanordnung (3b; 4b) umfasst, und in den Strohlengängen (3,4) Videochips (5,6) angeordnet sind und wobei das Verfahren die folgenden Schritte umfasst:
- Erfassen einer Benutzereingabe zum Einstellen einer Fokuseinstellung und eines Zoom-Faktors des stereomikroskops (2) mit Hilfe einer Eingabeeinrichtung (21);
- Erzeugen von der erfassten Benutzereingabe zugeordneten Steuersignalen zum Einstellen der Fokuseinstellung und des Zoom-Faktors in einer an die Eingabeeinrichtung (21) gekoppelten Steuereinrichtung (20), wobei die Steuersignale Vorabeinstellungsdaten entsprechend gebildet werden, die in einer mit der Steuereinrichtung (20) verbundenen Speichereinrichtung (22) vorab gespeichert sind;
- Übertragen der Steuersignale an die jeweiligen motorgetriebenen Verstelleinrichtungen (7-10, 13) zum Verlagern der ersten Linsenanordnung (3a; 4a), der zweiten Linsenanordnung (3b; 4b) und der Sammellinsenanordnung (11); und
- Einstellen der Fokuseinstellung und des Zoom-Faktors des Stereomikroskops (2) entsprechend den Steuersignalen, indem die erste Linsenanordnung (3a; 4a), die zweite Linsenanordnung (3b; 4b) und
die Sammellinsenanordnung (11) mit Hilfe der jeweiligen motorgetriebenen Verstelleinrichtung (7-10, 13) verlagert werden;
wobei
beim Einstellen der Fokuseinstellung eine grobe und eine feine Fokuseinstellung ausgeführt wird, indem
für die grobe Fokuseinstellung von den Steuersignalen umfasste Grobsteuersignale in der Steuereinrichtung (20) erzeugt und von der Steuereinrichtung (20) an die motorgetriebene Verstelleinrichtung (13) der Sammellinsenanordnung (11) übertragen werden und die Sammellinsenanordnung (11) mit Hilfe der motorgetriebenen Verstelleinrichtung (13) der Sammellinsenanordnung (11) den Grobsteuersignalen entsprechend verlagert wird; und
indem
für die feine Fokuseinstellung von den Steuersignalen umfasste Feinsteuersignale in der Steuereinrichtung (20) erzeugt und von der Steuereinrichtung (20) an die jeweilige motorgetriebene Verstelleinrichtung (7; 9) der ersten Linsenanordnung (3a; 4a) in einem oder allen Strahlengängen (3, 4) übertragen werden und die erste Linsenanordnung (3a, 4a) in einem oder allen Strahlengängen (3, 4) mit Hilfe der jeweiligen motorgetriebenen Verstelleinrichtung (7; 9) den Feinsteuersignalen entsprechend verlagert wird; und
wobei
das Einstellen des Zoom-Faktors ausgeführt wird, indem von den Steuersignalen umfasste Zoomsteuersignale in der Steuereinrichtung (20) erzeugt und von der Steuereinrichtung (20) an die jeweilige motorgetriebene Verstelleinrichtung (8; 10) der zweiten Linsenanordnung (3b; 4b) in einem oder allen Strahlengängen (3, 4) übertragen werden und die zweite Linsenanordnung (3b; 4b) in einem oder allen Strahlengängen (3, 4) mit Hilfe der jeweiligen motorgetriebenen Verstelleinrichtung (8; 10) den Zoomsteuersignalen entsprechend verlagert wird
wobei in den Strahlengängen (3, 4) ein jeweiliger Zoom-Faktor eingestellt wird, indem die zweite Linsenanordnung (3b; 4b)
mit Hilfe der jeweiligen motorgetriebenen Verstelleinrichtung (8;10) verlagert werden, wobei sich die jeweils einstellbaren Zoom-Faktoren der Strahlengänge (3, 4) voneinander unterscheiden.

2. Verfahren nach Anspruch 1, **dadurch gekennzeichnet, dass** beim Erzeugen der Steuersignale zum Einstellen der Fokuseinstellung und des Zoom-Faktors in der Steuereinrichtung (20) eine Interpolation zwischen ausgewählten Vorabeinstellungsdaten der gespeicherten Vorabeinstellungsdaten ausgeführt wird.

3. Verfahren nach einem der vorangehenden Ansprüche, **dadurch gekennzeichnet, dass** die Sammellinsenanordnung (11), die erste Linsenanordnung (3a; 4a) und / oder die zweite Linsenanordnung (3b; 4b) beim Verlagern entsprechend der Steuersignale mit Hilfe der jeweiligen motorgetriebenen Verstelleinrichtung (7-10, 13) linear verschoben werden.

4. Verfahren nach einem der vorangehenden Ansprüche, **dadurch gekennzeichnet, dass** mit den Steuersignalen in der jeweiligen motorgetriebenen Verstelleinrichtung (7-10, 13) ein Linearmotor beaufschlagt wird, welcher die den Steuersignalen entsprechende Verlagerung der Sammellinsenanordnung (11), der ersten Linsenanordnung (3a; 4a) und / oder der zweiten Linsenanordnung (3b; 4b) bewirkt.

5. Verfahren nach einem der vorangehenden Ansprüche, **dadurch gekennzeichnet, dass** es sich bei den motorgetriebenen Verstelleinrichtungen (7-10, 13) um elektromagnetische Wanderfeld-Linearmotoren handelt.

6. Verfahren nach einem der vorangehenden Ansprüche, **dadurch gekennzeichnet, dass** das Untersuchungsobjekt auf mehrere elektronische Speichermedien (5, 6), die jeweils einem Strahlengang (3; 4) zugeordnet sind, räumlich abgebildet wird, oder aber auch über Tuben direkt dem Betrachter zur Verfügung gestellt wird.

7. Optisches System (1) mit einem Stereomikroskop (2) zum Abbilden eines
Untersuchungsobjektes, wobei das Stereomikroskop (2) mindestens ein Linsensystem, welches in dem jeweiligen Strahlengang (3, 4) angeordnet ist, und eine in dem Strahlengängen (3, 4) angeordnete Sammellinsenanordnung (11) aufweist, und wobei das mindestens eine Linsensystem eine erste Linsenanordnung (3a; 4a) und eine zwischen der ersten Linsenanordnung (3a; 4a) und der Sammellinsenanordnung (11) angeordnete, zweite Linsenanordnung (3b; 4b) umfasst, und wobei das optische System (1) eine Eingabeeinrichtung (21) zum Erfassen einer Benutzereingabe für eine einzustellende Fokuseinstellung und einzustellende Zoom-Faktoren des Stereomikroskops (2) umfasst und wobei in den Strahlengängen (3, 4) Videochips (5, 6) angeordnet sind,
wobei das optische System **gekennzeichnet ist durch**:
- eine Steuereinrichtung (20), die an die Eingabeeinrichtung (21) gekoppelt ist und mit welcher der erfassten Benutzereingabe zugeordnete Steuersignale zum Einstellen der Fokuseinstellung und des Zoom-Faktors erzeugbar sind, wobei die Steuersignale Vorabeinstellungsdaten entsprechend gebildet werden, die in einer mit der Steuereinrichtung (20) verbundenen Speichereinrichtung (22) vorab gespeichert sind;
- eine jeweilige motorgetriebene Verstelleinrichtung (7-10, 13) der ersten Linsenanordnung (3a; 4a), der zweiten Linsenanordnung (3b; 4b) und der Sammellinsenanordnung (11), mit denen die erste Linsenanordnung (3a; 4a), die zweite Linsenanordnung (3b; 4b) und die Sammellinsenanordnung (11) den Steuersignalen entsprechend einstellbar sind; und
- Übertragungsmittel zum Übertragen der Steuersignale an die jeweiligen motorgetriebenen Verstelleinrichtungen (7- 10, 13) der ersten Linsenanordnung (3a; 4a), der zweiten Linsenanordnung (3b; 4b) und der Sammellinsenanordnung (11);
wobei
die motorgetriebene Verstelleinrichtung (13) der Sammellinsenanordnung (11) einer groben Folcuseinstellung zugeordnet ist, so dass mit der motorgetriebenen Verstelleinrichtung (13) der Sammellinsenanordnung (11) von der Steuereinrichtung (20) empfangene und von den Steuersignalen umfasste Grobsteuersignale verarbeitbar sind; und
wobei die motorgetriebene Verstelleinrichtung (7; 9) der ersten Linsenanordnung (3a; 4a) in einem oder allen Strahlengängen (3, 4) einer feinen Fokuseinstellung zugeordnet ist, so dass mit der motorgetriebenen Verstelleinrichtung (7; 9) der ersten Linsenanordnung (3a; 4a) in einem oder allen Strahlengängen (3, 4) von der Steuereinrichtung (20) empfangene und von den Steuersignalen umfasste Feinsteuersignale zum Einstellen der feinen Fokuseinstellung verarbeitbar sind; und wobei
mittels der jeweiligen motorgetriebenen Verstelleinrichtung (810) der zweiten Linsenanordnung (3b; 4b) in den Strahlengängen (3, 4) ein jeweiliger Zoom-Faktor einstellbar ist, wobei sich die jeweils einstellbaren Zoom-Faktoren der Strahlengänge (3, 4) voneinander unterscheiden
die motorgetriebene Verstelleinrichtung (8; 10) der zweiten Linsenanordnung (3b; 4b) in einem oder allen Strahlengängen (3, 4) dem Zoom-Faktor zugeordnet ist, so dass mit der motorgetriebenen Verstelleinrichtung (8; 10) der zweiten Linsenanordnung (3b; 4b) in einem oder allen Strahlengängen (3, 4) von der Steuereinrichtung (20) empfangene und von den Steuersignalen umfasste Zoomsteuersignale zum Einstellen des Zoom-Faktors verarbeitbar sind und wobei
mittels der jeweiligen motorgetriebenen Verstelleinrichtung (8,10)
der zweiten Linsenanordnung (3b,4b) in den Strahlengängen (3, 4) ein jeweiliger Zoom-Factor einstellbar ist, wobei sich die jeweils einstellbaren Zoom-Faktoren der Strahlengänge (3, 4) voneinander unterscheider.

8. Optisches System nach Anspruch 7, **dadurch gekennzeichnet, dass** die Steuereinrichtung (20) Interpolationsmittel umfasst, mit denen beim Erzeugen der Steuersignale zum Einstellen der Fokuseinstellung und / oder des Zoom-Faktors in der Steuereinrichtung (20) eine Interpolation zwischen ausgewählten Vorabeinstellungsdaten der gespeicherten Vorabeinstellungsdaten ausführbar ist.

9. Optisches System nach einem der Ansprüche 7 bis 8 **dadurch gekennzeichnet, dass** die jeweilige motorgetriebene Verstelleinrichtung (7-10, 13) der Sammellinsenanordnung (11), der ersten Linsenanordnung (3a; 4a) und / oder der zweiten Linsenanordnung (3b; 4b) einen Linearmotor umfasst.

10. Optisches System nach einem der Ansprüche 7 bis 9, **dadurch gekennzeichnet, dass** die Sammellinsenanordnung (11), die erste Linsenanordnung (3a; 4a) und / oder die zweite Linsenanordnung (3b; 4b) in
einer oder mehreren Hülsen angeordnet und mittels eines bewegten magnetischen Wanderfeldes in einer oder allen Hülsen jeweils verlagerbar sind.

11. Optisches System nach einem der Ansprüch 7 bis 10, **gekennzeichnet durch** mehrere elektronische Speichermedien (5, 6), die jeweils einem Strahlengang (3; 4) zugeordnet sind, zum Aufnehmen der räumlichen Abbildung des Untersuchungsobjektes.

12. Optisches System nach einem der Ansprüche 7 bis 11, **dadurch gekennzeichnet, dass** die jeweilige motorgetriebene Verstelleinrichtung (7-10, 13) der Sammellinsenanordnung (11), der ersten Linsenanordnung (3a; 4a) und / oder der zweiten Linsenanordnung (3b; 4b) drehfeldgetrieben sind.

## Claims

1. Method for the operation of an optical system (1), in which an object of study can be imaged spatially in an image plane by means of a stereomicroscope (2) comprised in the optical system, wherein the stereomicroscope (2) comprises at least one lens system, which is arranged in a respective optical path (3, 4) and which comprises a collecting lens arrangement (11) arranged in the optical paths (3, 4), wherein the at least one lens system comprises a first lens arrangement (3a; 4a) and a second lens arrangement (3b; 4b), which is positioned between the first lens arrangement (3a; 4a) and the collective lens arrangement (11), wherein video chips (5, 6) are arranged in the optical paths (3, 4), and wherein the method comprises the following steps:
- registering a user input for adjusting a focus setting and a zoom factor of the stereomicroscope (2) using an input device (21);
- generating controlling signals assigned to the registered user input for adjusting the focus setting and the zoom factor in a controlling device (20) coupled to the input device (21), wherein the controlling signals are built according to pre-settings data which are stored in advance in a storage device (22) connected to the controlling device (20);
- transmitting the controlling signals to the respective motor-powered adjustment devices (7-10, 13) for displacing the first lens arrangement (3a; 4a), the second lens arrangement (3b; 4b) and the collecting lens arrangement (11); and
- adjusting the focus setting and the zoom factor of the stereomicroscope (2) according to the controlling signals by displacing the first lens arrangement (3a; 4a), the second lens arrangement (3b; 4b) and the collecting lens arrangement (11) using the respective motor-powered adjustment devices (7-10, 13);
wherein during the adjustment of the focus setting a coarse and a fine focus adjustment is carried out
for the coarse focus adjustment by generating in the controlling device (20) coarse controlling signals comprised in the controlling signals and by transmitting them from the controlling device (20) to the motor-powered adjustment device (13) of the collecting lens arrangement (11) and by displacing the collecting lens arrangement (11) according to the coarse controlling signals using the motor-powered adjustment device (13) of the collecting lens arrangement (11); and
for the fine focus adjustment by generating in the controlling device (20) fine controlling signals comprised in the controlling signals and by transmitting them from the controlling device (20) to the motor-powered adjustment devices (7; 9) of the first lens arrangement (3a; 4a) in one or all optical paths (3; 4) and by displacing the first lens arrangement (3a; 4a) in one or all optical paths (3; 4) according to the fine controlling signals using the respective motor-powered adjustment devices (7; 9); and wherein
the adjustment of the zoom factor is carried out by generating in the controlling device (20) zoom controlling signals comprised in the controlling signals and by transmitting them from the controlling device (20) to the respective motor-powered adjustment devices (8; 10) of the second lens arrangement (3b; 4b) in one or all optical paths (3; 4) and by displacing the second lens arrangement (3b; 4b) in one or all optical paths (3, 4) according to the zoom controlling signals using the motor-powered adjustment devices (8; 10), wherein in the optical paths (3, 4) a respective zoom factor is adjusted by displacing the second lens arrangement (3b; 4b) using the respective motor-powered adjustment devices (8; 10), wherein the in each case adjustable zoom factors of the optical paths (3, 4) differ from each other.

2. Method according to claim 1, **characterised in that** during the generation of the controlling signals for the adjustment of the focus setting and the zoom factor in the controlling device (20), an interpolation between selected pre-settings data of the stored pre-settings data is carried out.

3. Method according to any of the preceding claims, **characterised in that** the collecting lens arrangement (11), the first lens arrangement (3a; 4a) and/or the second lens arrangement (3b; 4b) is linearly displaced during the relocation according to the controlling signals using the respective motor-powered adjustment device(s) (7-10; 13).

4. Method according to any of the preceding claims, **characterised in that** a linear motor, which effects the displacement of the collecting lens arrangement (11), of the first lens arrangement (3a; 4a) and/or of the second lens arrangement (3b; 4b) according to the controlling signals, is acted upon by the controlling signals in the respective motor-powered adjustment device(s) (7-10, 13).

5. Method according to any of the preceding claims, **characterised in that** the motor-powered adjustment devices (7-10, 13) are electromagnetic gliding field linear motors.

6. Method according to any of the preceding claims, **characterised in that** the object of study is spatially imaged onto several electronic storage media (5, 6) which are assigned to one optical path each (3, 4) or is also made directly available to the observer via tubes.

7. Optical system (1) with a stereomicroscope (2) for imaging an object of study, wherein the stereomicroscope (2) comprises at least one lens system each arranged in a respective optical path (3, 4) and a collecting lens arrangement (11) arranged in the optical paths (3, 4), wherein the at least one lens system comprises a first lens arrangement (3a; 4a) and a second lens arrangement (3b; 4b), which is positioned between the first lens arrangement (3a; 4a) and the collective lens arrangement (11), wherein the optical system (1) comprises an input device (21) for registering a user input for a stereomicroscope (2) focus setting and zoom factors both to be adjusted, and wherein video chips (5, 6) are arranged in the optical paths (3, 4);
wherein the optical system is **characterised by**:
- a controlling device (20) which is coupled to an input device (21) and with which controlling signals for adjusting the focus setting and the zoom factor are producible, said controlling signals being assigned to the registered user input, wherein the controlling signals are generated according to pre-settings data, which are stored in advance in a storage device (22) connected with the controlling device (20);
- a respective motor-powered adjustment device (7-10; 13) of the first lens arrangement (3a; 4a), of the second lens arrangement (3b; 4b) and of the collecting lens arrangement (11), with which the first lens arrangement (3a; 4a), the second lens arrangement (3b; 4b) and the collecting lens arrangement (11) are adjustable according to the controlling signals; and
- transmitting devices for transmitting the controlling signals to the respective motor-powered adjustment devices (7-10, 13) of the first lens arrangement (3a; 4a), the second lens arrangement (3b; 4b) and the collecting lens arrangement (11);
wherein
the motor-powered adjustment device (13) of the collecting lens arrangement (11) is assigned to a coarse focus adjustment so that coarse controlling signals comprised in the controlling signals and received from the controlling device (20) are processible with the motor-powered adjustment device (13) of the collecting lens arrangement (11);
wherein the motor-powered adjustment device (7; 9) of the first lens arrangement (3a; 4a) in one or all optical paths (3, 4) is assigned to a fine focus adjustment, so that fine controlling signals, which are comprised in the controlling signals and received from the controlling device (20), for adjusting the fine focus setting are processible with the motor powered adjustment device (7; 9) of the first lens arrangement (3a; 4a) in one or all optical paths (3, 4);
wherein
the motor-powered adjustment device (8; 10) of the second lens arrangement (3b; 4b) in one or all optical paths (3, 4) is assigned to the zoom factor so that zoom controlling signals, which are comprised in the controlling signals and received from the controlling device (20), for adjusting the zoom factor are processible with the motor-powered adjustment device (8; 10) of the second lens arrangement (3b; 4b) in one or all optical paths (3, 4); and
wherein in the optical paths (3, 4) a respective zoom factor is adjustable via the respective motor-powered devices (8; 10) of the second lens arrangement (3b; 4b), wherein the in each case adjustable zoom factors of the optical paths (3, 4) differ from each other.

8. Optical system according to claim 7, **characterised in that** the controlling device (20) comprises interpolation means with which during the generation of controlling signals for adjusting focus setting and/or zoom factor in the controlling device (20), an interpolation can be carried out between selected pre-settings data of the stored pre-settings data.

9. Optical system according to any one of claims 7 to 8, **characterised in that** the respective motor-powered adjustment device(s) (7-10, 13) of the collecting lens arrangement (11), of the first lens arrangement (3a; 4a) and/or of the second lens arrangement (3b; 4b) comprises a linear motor.

10. Optical system according to any one of claims 7 to 9, **characterised in that** the collecting lens arrangement (11), the first lens arrangement (3a; 4a) and/or the second lens arrangement (3b; 4b) are arranged in one or more sleeves and that they are each displaceable using a moveable magnetic gliding field in one or all sleeves.

11. Optical system according to any one of claims 7 to 10, **characterised by** several electronic storage media (5, 6) which are assigned to an optical path each (3; 4) for receiving the spatial image of the object of study.

12. Optical system according to any one of claims 7 to 11, **characterised in that** the respective motor-powered adjustment device (7-10, 13) of the collecting lens arrangement (11), of the first lens arrangement (3a; 4a) and/or of the second lens arrangement (3b; 4b) are powered by a revolving field.

## Revendications

1. Procédé pour l'exploitation d'un système optique (1) dans lequel on peut représenter dans l'espace au moyen d'un stéréomicroscope (2) compris dans le système optique (1) un objet d'investigation dans un plan image, tandis que le stéréomicroscope (2) présente un système de lentilles qui est situé dans un trajet de rayons (3, 4) respectivement concerné, et un arrangement de lentilles de convergence (11) disposées dans les trajets des rayons (3, 4), tandis que le au moins un système de lentilles comporte un premier arrangement de lentilles (3a; 4a) et un deuxième arrangement de lentilles (3b; 4b) placé entre le premier arrangement de lentilles (3a; 4a) et l'arrangement de lentilles de convergence (11), et que dans les trajets de rayons (3, 4) sont disposées des vidéopuces (5, 6), tandis que le procédé comporte les étapes suivantes:
- enregistrement d'une donnée utilisateur pour l'ajustement d'une focale et d'un facteur de zoom du stéréomicroscope (2) au moyen d'un dispositif d'introduction (21);
- déclenchement de signaux de commande affectés à la donnée utilisateur enregistrée pour l'établissement de l'ajustement de la focale et du facteur de zoom dans une installation de commande (20) couplée au dispositif d'introduction (21), tandis que les signaux de commande sont formés en correspondance avec des données d'ajustement initial qui sont initialement mémorisées dans une unité de mémoire (22) reliée à l'installation de commande (20);
- transfert des signaux de commande dans les dispositifs d'ajustement motorisés respectifs (7-10, 13) pour le déplacement du premier arrangement de lentilles (3a; 4a), du deuxième arrangement de lentilles (3b; 4b), et de l'arrangement de lentilles de convergence (11); et
- ajustement du réglage de focale et du facteur de zoom du stéréomicroscope (2) en fonction des signaux de commande, tandis que le premier arrangement de lentilles (3a; 4a), le deuxième arrangement de lentilles (3b; 4b) et l'arrangement de lentilles de convergence (11) sont déplacés au moyen du dispositif d'ajustement motorisé (7-10, 13) respectif;
tandis que
lors de l'ajustement du réglage de focale est effectué un ajustement grossier et un ajustement fin de la focale,
alors que
pour l'ajustement grossier de la focale, des signaux de commande grossière compris dans les signaux de commande sont produits dans le dispositif de commande (20) et sont transférés du dispositif de commande (20) au dispositif d'ajustement motorisé (13) de l'arrangement de lentilles de convergence (11), et l'arrangement de lentilles de convergence (11) est déplacé en fonction des signaux de commande grossière au moyen du dispositif d'ajustement motorisé (13) de l'arrangement de lentilles de convergence (11); et
alors que
pour l'ajustement fin de la focale, des signaux de commande fine compris dans les signaux de commande sont produits dans le dispositif de commande (20), et sont transférés du dispositif de commande (20) au dispositif d'ajustement motorisé respectivement concerné (7; 9) du premier arrangement de lentilles (3a; 4a) dans l'un ou la totalité des trajets de rayons (3, 4), et le premier arrangement de lentilles (3a, 4a) dans l'un ou la totalité des trajets de rayons (3, 4) est déplacé en fonction des signaux de commande fine au moyen du dispositif d'ajustement motorisé respectivement concerné (7; 9); et tandis que
l'ajustement du facteur de zoom est effectué par production de signaux de commande de zoom compris dans les signaux de commande dans le dispositif de commande (20) et leur transfert du dispositif de commande (20) au dispositif de déplacement motorisé concerné (8; 10) du deuxième arrangement de lentilles (3b; 4b) dans l'un ou la totalité des trajets de rayons (3, 4), et le deuxième dispositif de lentilles (3b; 4b) est déplacé en fonction des signaux de commande de zoom dans l'un ou la totalité des trajets de rayons (3, 4) au moyen du dispositif de déplacement motorisé respectivement concerné (8; 10),
tandis que dans les trajets de rayons (3, 4) est ajusté un facteur de zoom respectif, alors que le deuxième arrangement de lentilles (3b; 4b) est déplacé au moyen du dispositif de déplacement motorisé respectivement concerné (8; 10), tandis que les facteurs de zoom à chaque fois ajustables des trajets de rayons (3, 4) sont différents les uns des autres.

2. Procédé selon la revendication 1, **caractérisé en ce que** lors de la production des signaux de commande pour l'ajustement du réglage de la focale et du facteur de zoom dans le dispositif de commande (20) on effectue une interpolation entre des données de préréglage sélectionnées des données de préréglage mémorisées.

3. Procédé selon l'une quelconque des revendications précédentes, **caractérisé en ce que** l'arrangement de lentilles de convergence (11), le premier arrangement de lentilles (3a; 4a) et/ou le deuxième arrangement de lentilles de convergence (3b; 4b) sont décalés de manière linéaire lors du déplacement en fonction des signaux de commande au moyen du dispositif de déplacement motorisé respectivement concerné (7-10,13).

4. Procédé selon l'une quelconque des revendications précédentes, **caractérisé en ce que**, avec les signaux de commande dans le dispositif d'ajustement motorisé concerné (7-10, 13) est admis un moteur linéaire qui effectue le déplacement correspondant aux signaux de commande, de l'arrangement de lentilles de convergence (11), du premier arrangement de lentilles (3a; 4a) et/ou du deuxième arrangement de lentilles (3b; 4b).

5. Procédé selon l'une quelconque des revendications précédentes, **caractérisé en ce qu'**il s'agit, en ce qui concerne les dispositifs d'ajustement motorisés (7-10, 13), de moteurs linéaires électromagnétiques à champ d'ondes progressives.

6. Procédé selon l'une quelconque des revendications précédentes, **caractérisé en ce que** l'objet d'investigation est représenté dans l'espace sur plusieurs moyens de mémorisation électroniques (5, 6) qui sont à chaque fois affectés à un trajet de rayons (3; 4), ou encore est mis directement à la disposition de l'observateur par l'intermédiaire de tubes.

7. Système optique (1) ayant un stéréomicroscope (2) pour la représentation d'un objet d'investigation, dans lequel le stéréomicroscope (2) possède au moins un système de lentilles qui est placé dans le trajet de rayons à chaque fois concerné (3, 4), et un arrangement de lentilles de convergence (11) placé dans les trajets de rayons (3, 4), et tandis que le au moins un système de lentilles comprend un premier arrangement de lentilles (3a; 4a) et un deuxième arrangement de lentilles (3b; 4b) disposé entre le premier arrangement de lentilles (3a; 4a) et l'arrangement de lentilles de convergence (11), et tandis que le système optique (1) comporte un dispositif d'introduction (21) pour la saisie d'une donnée d'utilisateur pour l'ajustement de la focale à ajuster et des facteurs de zoom du stéréomicroscope (2) à ajuster, et tandis que des vidéopuces (5, 6) sont placées dans les trajets de rayons (3, 4);
tandis que le système optique est **caractérisé par**:
- un dispositif de commande (20) qui est couplé au dispositif d'introduction (21) et avec lequel des signaux de commande attribués à la donnée d'utilisateur détectée peuvent être générés pour l'ajustement du réglage de la focale et du facteur de zoom, tandis que les signaux de commande sont formés en fonction des données de préréglage, qui sont préalablement mémorisées dans un dispositif de mémorisation (22) relié au dispositif de commande (20);
- un dispositif de réglage à chaque fois motorisé (7-10, 13) du premier arrangement de lentilles (3a; 4a), du deuxième arrangement de lentilles (3b; 4b) et de l'arrangement de lentilles de convergence (11), avec lesquels le premier arrangement de lentilles (3a; 4a), le deuxième arrangement de lentilles (3b; 4b) et l'arrangement de lentilles de convergence (11) peuvent être ajustés en fonction des signaux de commande; et
- des moyens de transmission pour la transmission des signaux de commande aux dispositifs d'ajustement respectivement concernés (7-10, 13) du premier arrangement de lentilles (3a; 4a), du deuxième arrangement de lentilles (3b; 4b) et de l'arrangement de lentilles de convergence (11);
tandis que
le dispositif d'ajustement motorisé (13) de l'arrangement de lentilles de convergence (11) est affecté à un ajustement grossier de la focale, de telle sorte que l'on peut traiter avec le dispositif d'ajustement motorisé (13) de l'arrangement de lentilles de convergence (11) des signaux de commande grossière reçus par le dispositif de commande (20) et compris dans les signaux de commande; et
tandis que le dispositif d'ajustement motorisé (7; 9) du premier arrangement de lentilles (3a; 4a) est affecté à un ajustement fin de la focale dans l'un ou la totalité des trajets des rayons (3, 4), de telle sorte que l'on peut traiter avec le dispositif d'ajustement motorisé (7; 9) du premier arrangement de lentilles (3a; 4a) des signaux de commande fine reçus dans un ou la totalité des trajets des rayons (3, 4) reçus par le dispositif de commande (20) et compris dans les signaux de commande, pour l'ajustement du réglage fin de la focale ; et
tandis que
le dispositif d'ajustement motorisé (8; 10) du deuxième arrangement de lentilles (3b; 4b) est affecté au facteur de zoom dans l'un ou la totalité des trajets des rayons (3, 4), de telle sorte que l'on peut traiter avec le dispositif d'ajustement motorisé (8; 10) du deuxième arrangement de lentilles (3b; 4b) des signaux de commande de zoom reçus dans un ou la totalité des trajets des rayons (3,4) reçus par le dispositif de commande (20) et compris dans les signaux de commande, pour l'ajustement du réglage du facteur de zoom, et tandis que, au moyen du dispositif d'ajustement motorisé respectif concerné (8; 10) du deuxième arrangement de lentilles (3b; 4b) dans les trajets des rayons (3, 4) on peut ajuster un facteur de zoom particulier, tandis que les facteurs de zoom des trajets de rayons (3, 4) respectivement ajustables sont différents les uns des autres.

8. Système optique selon la revendication 7, **caractérisé en ce que** le dispositif de commande (20) comprend des moyens d'interpolation, avec lesquels lors de la production des signaux de commande pour l'ajustement du réglage de la focale et/ou du facteur de zoom dans le dispositif de commande (20) on peut effectuer une interpolation entre des données de préréglage sélectionnées des données de préréglage mémorisées.

9. Système optique selon l'une des revendications 7 à 8, **caractérisé en ce que** le dispositif d'ajustement motorisé respectif concerné (7-10, 13) de l'arrangement de lentilles de convergence (11), du premier arrangement de lentilles (3a; 4a) et/ou du deuxième arrangement de lentilles (3b; 4b) comprend un moteur linéaire.

10. Système optique selon l'une quelconque des revendications 7 à 9, **caractérisé en ce que** l'arrangement de lentilles de convergence (11), le premier arrangement de lentilles (3a; 4a) et/ou le deuxième arrangement de lentilles (3b; 4b) peuvent être disposés dans une ou plusieurs gaines et être déplacés à chaque fois au moyen d'un champ magnétique mobile à ondes progressives, dans l'une ou dans la totalité des gaines.

11. Système optique selon l'une quelconque des revendications 7 à 10, **caractérisé par** plusieurs moyens de mémorisation électroniques (5, 6), qui sont à chaque fois affectés à un trajet de rayons (3; 4), pour l'enregistrement de la représentation spatiale de l'objet des investigations.

12. Système optique selon l'une quelconque des revendications 7 à 11, **caractérisé en ce que** le dispositif d'ajustement motorisé respectif concerné (7-10, 13) de l'arrangement de lentilles de convergence (11), du premier arrangement de lentilles (3a; 4a) et/ou du deuxième arrangement de lentilles (3b; 4b) sont entraînés par un champ rotatif.
